# EUROPÄISCHE PATENTSCHRIFT

(11) **EP 3 254 709 B1**
(45) Veröffentlichungstag und Bekanntmachung des Hinweises auf die Patenterteilung: **26.02.2020**
(21) Anmeldenummer: 17162233.5
(22) Anmeldetag: 22.03.2017
(51) Int. Cl.: A61L 24/04, A61L 24/06, A61L 27/26, A61F 2/28, C08L 33/12

(54) **PASTENFÖRMIGER ZWEIKOMPONENTEN-POLYMETHACRYLAT-KNOCHENZEMENT**
TWO COMPONENT POLYMETHACRYLATE BONE CEMENT IN PASTE FORM
CIMENT OSSEUX EN POLYMÉTHACRYLATE DE MÉTHYLE BICOMPOSANT SOUS FORME DE PÂTE

(30) Priorität: 07.06.2016 DE 102016209988
(43) Veröffentlichungstag der Anmeldung: 13.12.2017
(73) Patentinhaber: Heraeus Medical GmbH, 61273 Wehrheim (DE)
(72) Erfinder: VOGT, Sebastian, 99092 Erfurt (DE); KLUGE, Thomas, 56179 Vallendar (DE)
(74) Vertreter: Heraeus IP

(56) Entgegenhaltungen:
- WO-A1-2015/044689
- DE-A1- 1 567 481
- DE-A1-102009 043 550
- DE-B3-102007 050 762

## Beschreibung

Gegenstand der Erfindung ist ein pastenförmiger Zweikomponenten-Polymethacrylat-Knochenzement. Weiterhin sind ein Verfahren zur Herstellung eines selbsthärtenden Knochenzementteigs und die Verwendung des pastenförmigen Zweikomponenten-Polymethacrylat-Knochenzements Gegenstand der Erfindung.

In der Medizin werden seit Jahrzehnten Polymethacrylat-Knochenzemente zur dauerhaften mechanischen Fixierung von Totalgelenkendoprothesen eingesetzt. Diese basieren auf Pulver-Flüssigkeit-Systemen, wobei Methylmethacrylat üblicherweise als Monomer verwendet wird. Ein allgemeiner Überblick findet sich z. B. in K.-D. Kühn, "Bone cements: Up-to-date Comparison of Physical and Chemical Properties of Commercial Materials", Springer-Verlag Berlin Heidelberg New York, 2000.

Neben den Pulver-Flüssigkeit-Zementen wurden auch Polymethylmethacrylat-Knochenzemente vorgeschlagen, die auf der Verwendung von Zementpasten beruhen. DE 10 2007 052116 A1 betrifft einen Einkomponenten-Knochenzement. DE 32 45 956 A1, DE 10 2007 050762 B3 und DE 10 2008 030312 A1 beschreiben Zweikomponenten-Knochenzemente aus zwei Zementpasten, die separat in geeigneten Kartuschen gelagert werden. Diese Pasten enthalten immer ein Methacrylat-Monomer und mindestens ein darin gelöstes Polymer. Weiterhin können diese Pasten noch im Methacrylat-Monomer unlösliche, vernetzte Polymerpartikel enthalten. In den beiden pastenförmigen Komponenten sind Bestandteile von Redoxinitiatorsystemen separat enthalten, die erst nach der Vermischung der beiden pastenförmigen Komponenten unter Bildung von Radikalen reagieren, welche die radikalische Polymerisation des Methacrylat-Monomeren auslösen, die zur Aushärtung des gemischten Zementteigs führt. Besonders geeignet sind Redoxinitiatorsysteme, die auf thermisch stabilen Hydroperoxiden beruhen. In EP 2 664 349 B1 wird ein Pastenzement mit einem Redoxinitiatorsystem beschrieben, das auf der Verwendung von Hydroperoxiden beruht.

Das seit Jahrzehnten bewährte Redoxinitiatorsystem Dibenzoylperoxid/N,N-p-Toluidin ist bisher für pastenförmige Zweikomponenten-Polymethacrylat-Knochenzemente nur bedingt geeignet, weil im flüssigen Monomer gelöstes Dibenzoylperoxid immer einen geringen Spontanzerfall zeigt, der die Lagerfähigkeit der Zementpasten zeitlich begrenzt. Im festen, ungelösten Aggregatszustand ist das Dibenzoylperoxid deutlich lagerungsstabiler als im gelösten Zustand.

US 8,536,243 B2 beschreibt ein Pulver-Gel-Knochenzement-System. Bei diesem handelt es sich um eine Modifikation der konventionellen Pulver-Flüssigkeit-Knochenzemente. Die pulverförmige Komponente besteht aus einem Polymer, einem Initiator, wie Dibenzoylperoxid, und gegebenenfalls einem Röntgenopaker und Hydroxylapatit. Die gelartige Komponente umfasst ein Acrylmonomer, wie Methylmethacrylat, einen Radikalinhibitor, wie Hydrochinon, einen Aktivator, wie N,N-Dimethyl-p-toluidin, und ein im Acrylmonomer gelöstes Polymer mit einem mittleren Molekulargewicht von größer 1 000 000 g/mol. Bei diesem Zement liegt das Dibenzolyperoxid als Feststoff im Zementpulver vor.

Ein analoges Knochenzementsystem wird in US 2011 270 259 A1 offenbart.

DE 10 2007 015698 B4 beschreibt ein Implantatmaterial. Dieses besteht aus einer lagerstabilen Paste und einer separat dazu gelagerten Monomerflüssigkeit. Bei der lagerstabilen Paste handelt es sich um ein Gemisch aus mindestens einem partikulärem Polymer, einer Starterkomponente für die radikalische Polymerisation und einer Trägerflüssigkeit, in der das partikuläre Polymer nicht löslich ist und auch nicht aufquillt und in der die Starterkomponente unlöslich ist. Es sind relativ große Gewichtsanteile an Trägerflüssigkeit notwendig, um das gesamte Zementpulver zu benetzen und in einen pastenförmigen Zustand zu überführen. Als separate Monomerflüssigkeit werden Methylmethacrylat oder andere Ester der Methacrylsäure vorgeschlagen. Als Starterkomponente wird Dibenzoylperoxid verwendet. Die Druckfestigkeit des ausgehärteten Implantatmaterials ist relativ gering und unterschreitet die Vorgaben der ISO5833 für die Druckfestigkeit von größer, gleich 70 MPa, wie das Beispiel 3 von DE 10 2007 015698 A1 veranschaulicht. Der Grund wird darin vermutet, dass die Trägerflüssigkeit nicht mit der Monomerflüssigkeit mischbar ist und auch nicht an der radikalischen Polymerisation teilnimmt. In diesem Fall entsteht ein poröses Implantatmaterial, das nur eine geringe mechanische Festigkeit besitzt. Damit ist die Verwendung dieses Implantatmaterials zur lasttragenden dauerhaften Verankerung von Gelenkendoprothesen kaum geeignet.

DE 10 2009 043550 A1 beschreibt einen Knochenzement, welcher aus einer hydrophilen und einer hydrophoben Komponente, aufweisend ein biodegradierbares Material, wie beispielsweise Hydroxylapatit, aufgebaut ist.

Die Erfindung hat die Aufgabe, einen pastenförmigen Zweikomponenten-Polymethacrylat-Knochenzement zu entwickeln, der als Initiator Dibenzoylperoxid enthält. Dieser Knochenzement soll aus zwei pastenförmigen Komponenten A und B aufgebaut sein, wobei die pastenförmige Komponente A einen Akzelerator enthält, und die pastenförmigen Komponente B den Initiator enthält und so zusammengesetzt sein soll, dass sie bei Raumtemperatur lagerungsstabil ist. Wichtig ist, dass die pastenförmige Komponente B als eine unter Druckbeanspruchung fließfähige Masse vorliegen soll, die mit konventionellen Pastenabfüllmaschinen zur Befüllung von Kartuschen geeignet ist.

Weiterhin ist es eine Aufgabe der Erfindung einen pastenförmigen Zweikomponenten-Polymethacrylat-Knochenzement bereit zu stellen, dessen ausgehärteter Zementteig nach zuvor erfolgter Vermischung der beiden pastenförmigen Zementkomponenten die Anforderungen der ISO5833 von einer Druckfestigkeit von größer gleich 70 MPa, einer Biegefestigkeit von größer gleich 50 MPa und einem Biegemodul von größer gleich 1800 MPa erfüllt.

Die Aufgabe der Erfindung wird durch einen pastenförmigen Zweikomponenten-Polymethacrylat-Knochenzement gemäß dem Anspruch 1 gelöst.

Erfindungsgemäß umfasst ein pastenförmiger Zweikomponenten-Polymethacrylat-Knochenzement eine pastenförmige Komponente A und eine pastenförmige Komponente B. Die pastenförmige Komponente A enthält AI mindestens ein destillierbares, radikalisch polymerisierbares Methacrylatmonomer, AII mindestens ein in AI lösliches Polymer, AIII mindestens ein in AI nicht lösliches partikuläres Polymer mit einer Partikelgröße von höchstens 500 µm, AIV mindestens einen radikalischen Stabilisator und AV mindestens einen Akzelerator aus der Gruppe der aromatischen Amine. Die pastenförmige Komponente B enthält nur BI Dibenzoylperoxid und BII mindestens eine nicht radikalisch polymerisierbare, bei Raumtemperatur flüssige Substanz, in der Dibenzoylperoxid eine Löslichkeit kleiner 5,0 Gewichtsprozent bei Raumtemperatur hat. Dabei ist nach Vermischung der pastenförmigen Komponente A mit der pastenförmigen Komponente B in dem gebildeten selbsthärtenden Zementteig der Gewichtsanteil der flüssigen Substanz BII kleiner 2,0 Gewichtsprozent.

Der Ausdruck "umfassen", wie er hier in der vorliegenden Erfindung verwendet, schließt neben "enthalten" auch "bestehen aus" und "im Wesentlichen bestehen aus" mit ein. Der Ausdruck "enthalten" schließt gleichermaßen "bestehen aus" und "im Wesentlichen bestehen aus" mit ein.

Unter Partikelgröße D50 wird hier das Volumenmittel der Partikelgröße verstanden. D50 wird über ein Laserbeugungsverfahren mittels Streulichtbestimmung mit dem Laser Diffraction Particle Size Analyzer LS 13320 der Firma Beckman Coulter, USA, bestimmt.

Die zahlenmittlere Molmasse wird mittels Gelpermeationschromatographie (GPC) bestimmt.

Der Ausdruck "Methacrylat" im Sinne dieser Erfindung bedeutet Alkylester der Methacrylsäure. Bevorzugt sind Ester ein- und zweiwertiger Alkohole mit 1 bis 4 Kohlenstoffatomen. Beispiele sind Methylmethacrylat und Ethylenglykolmethacrylat.

Der Ausdruck "Polymethacylat" bedeutet Polymer eines Alkylesters der Methacrylsäure. Bevorzugt sind Polymere des Methyl- und des Ethylesters, d.h. Polymethylmethacrylat und Polyethylmethacrylat, insbesondere Polymethylmethacrylat.

Der Ausdruck "in Methacrylat löslich" bedeutet hierbei, dass eine sichtklare Lösung gebildet wird. Dies wird visuell mit dem Auge bestimmt.

Bezüglich des in Methacrylat unlöslichen, partikulären Polymers, das eine Partikelgröße D50 von höchstens 500 µm, bedeutet "in Methacrylat unlöslich", dass keine sichtklare Lösung gebildet wird. Dies wird visuell mit dem Auge bestimmt.

Die Erfindung basiert auf der überraschenden Tatsache, dass im Gegensatz zum in der DE 10 2007 015698 B4 beschriebenen Knochenzement die pastenförmige Komponente B nur aus Dibenzoylperoxid und einer nicht radikalisch polymerisierbaren, bei Raumtemperatur flüssigen Substanz, in der Dibenzoylperoxid eine Löslichkeit kleiner 5,0 Gewichtsprozent bei Raumtemperatur hat, herstellbar ist, ohne dass polymere Partikel oder in einem Lösungsmittel gelöste Polymere zur Pastenbildung erforderlich sind. Überraschend ist dabei, dass diese Paste problemlos plastisch verformt werden kann und in gleicher Weise wie Pasten, die gelöste oder suspendierte Polymere enthalten, verarbeitet werden kann. Diese Paste kann problemlos mit Pasten vermischt werden, die Methacrylatmonomere enthalten, in denen Dibenzoylperoxid löslich ist. Es wurde überraschend gefunden, dass eine Vermischung der pastenförmigen Komponente B mit der pastenförmige Komponente A sehr einfach erfolgen kann, im Gegensatz zu einer Vermischung von zwei pastenförmigen Komponenten, die beide gelöste Polymere enthalten.

Überraschend wurde auch gefunden, dass bei dem erfindungsgemäßen Zweikomponenten-Polymethacrylat-Knochenzement nach Vermischung der pastenförmigen Komponenten A und B ein Zementteig erhalten wird, der nach seiner Aushärtung die Anforderungen der ISO5833 hinsichtlich der Druckfestigkeit von größer, gleich 70 MPa, der Biegefestigkeit von größer, gleich 50 MPa und des Biegemoduls von größer, gleich 1800 MPa erfüllt.

Die pastenförmigen Komponenten A und B können in Kartuschen aus Kunststoff oder in beschichteten Metallkartuschen, insbesondere lackierten Aluminiumkartuschen gelagert werden. Die Vermischung der pastenförmigen Komponenten A und B zu einem klebfreien Zementteig kann mit einem statischen Mischer vorgenommen werden, der in einem Austragsrohr angeordnet ist, das mit den Kartuschen am Kartuschenkopf verbunden ist. Die Kartuschen können als koaxiale Kartuschen, als Side-by-Side-Kartuschen oder auch als nicht miteinander verbundene, einzelne Kartuschen vorliegen, in denen die pastenförmigen Komponenten A und B separat vor der Vermischung aufbewahrt werden.

Die als Komponente A eingesetzte Paste umfasst AI mindestens ein destillierbares, radikalisch polymerisierbares Monomer. Beispiele für derartige Monomere sind insbesondere Ester der Methacrylsäure. Als Methacrylatmonomer werden Methylmethacrylat und Ethylenglykoldimethacrylat bevorzugt.

Besonders bevorzugt ist Methylmethacrylat als destillierbares radikalisch polymerisierbares Monomer. Der Gewichtsanteil des Monomeren in der Komponente A kann in breiten Bereichen variieren, wobei der Gewichtsanteil bevorzugt im Bereich von 10 bis 70 Gew.-% liegt. Der Gewichtsanteil von MMA in der Komponente A liegt bevorzugt im Bereich von 30 bis 50 Gew.-%.

Die als Komponente A eingesetzte Paste umfasst ferner All mindestens ein in dem destilierbaren, radikalisch polymerisierbaren Monomer lösliches Polymer. Es können prinzipiell alle in Methylmethacrylat löslichen Polymere verwendet werden. Es können ein oder mehrere in Methylmethacrylat lösliche Polymere verwendet werden. Beispiele für geeignete Polymere sind Polymethylmethacrylat und Copolymere von Methylmethacrylat mit einem oder mehreren damit copolymerisierbaren Monomeren, wie Methylacrylat, Styren und Ethylacrylat. Als Polymer AII werden Polymethylmethacrylat, Poly-methylmethacrylatcopolymere oder Mischungen aus diesen bevorzugt, wobei Polymethylmethacrylat-co-methylacrylat oder Polymethylmethacrylat-co-styren als Polymer besonders bevorzugt sind.

Vorzugsweise hat das lösliche Polymer eine zahlenmittlere Molmasse kleiner 500.000 Dalton. Die zahlenmittlere Molmasse des mindestens einen in Methacrylat löslichen Polymers beträgt bevorzugt mindestens 100.000 Dalton.

Der Gewichtsanteil von dem AII mindestens ein in Methacrylat lösliches Polymer in der Komponente A kann in breiten Bereichen variieren. Er kann beispielsweise im Bereich von 15 bis 45 Gew.-% liegen, wobei der Gewichtsanteil bevorzugt im Bereich von 25 bis 35 Gew.-% liegt.

Die als Komponente A eingesetzte Paste umfasst ferner mindestens ein in Methacrylat unlösliches, partikuläres Polymer. Das in Methacrylat unlösliche partikuläre Polymer weist vorzugsweise eine zum Methacrylat nur geringfügig abweichende Dichte auf. Dadurch tritt eine Sedimentation der unlöslichen Polymerpartikel in der Paste A kaum bzw. praktisch nicht auf.

Als in Methacrylat unlösliche Polymerteilchen können prinzipiell alle in Methacrylat unlösliche Polymere verwendet werden. Beispiele für geeignete Polymere sind vernetztes Polymethylmethacrylat, vernetztes Poly(methylmethacrylat-co-methacrylat) und vernetztes Poly(methylmethacrylat-co-styren).

Vernetztes Polymethylmethacrylat und vernetztes Poly(methylmethacrylat-co-methacrylat) stellen ein Copolymer von Methylmethacrylat oder von einer Mischung von Methylmethacrylat und Methacrylat mit einem oder mehreren damit copolymerisierbaren difunktionellen, trifunktionellen und/oder mehrfachfunktionellen Monomeren dar. Beispiele für die als Vernetzer fungierenden difunktionellen, trifunktionellen und mehrfachfunktionellen Monomeren sind Dimethacrylate, Trimethacrylate, Tetramethacrylate, Diacrylate, Triacrylate, Tetraacrylate, wobei Dimethacrylate bevorzugt sind.

Das in Methacrylat unlösliche, partikuläre Polymer ist bevorzugt ausgewählt aus der Gruppe, die aus vernetztem Polymethylmethacrylat und vernetzten Copolymeren von Methylmethacrylat besteht. Besonders bevorzugt ist AIII das in Methacrylat unlösliche, partikuläre Polymer ausgewählt aus vernetztem Polymethylmethacrylat und vernetztem Polymethylmethacrylat-co-methylacrylat.

Der Gewichtsanteil von dem AIII mindestens ein in Methacrylat unlösliches partikuläres Polymer in der Komponente A kann in breiten Bereichen variieren. Er kann beispielsweise im Bereich von 15 bis 45 Gew.-% liegen, wobei der Gewichtsanteil bevorzugt im Bereich von 25 bis 35 Gew.-% liegt.

Erfindungsgemäß sind in der pastenförmigen Komponente A 30-50 Gewichtsteile (AI), 25-35 Gewichtsteile (AII) und 25-35 Gewichtsteile (AIII) besonders bevorzugt vorhanden. Besonders bevorzugt ist das Gewichtsverhältnis der pastenförmigen Komponente A zur pastenförmigen Komponente B im Gewichtsverhältnis größer gleich 96 zu 4 ist, besonders bevorzugt liegt das Gewichtsverhältnis von Komponente A zu Komponente B zwischen 94 : 6 und 99,9 zu 0,1.

Die pastenförmige Komponente A wird unter Ausschluß von Luftsauerstoff in Kartuschen gelagert. Sie enthält AIV einen radikalischen Stabilisator. Dieser radikalische Stabilisator wird vorteilhaft aus der Gruppe ausgewählt, die aus p-Benzochinon (CAS-Nr. 106-51-4), o-Benzochinon (CAS-Nr. 583-63-1), Hydrochinon (CAS-Nr.123-31-9), 2,5-Di-t-butylhydrochinon (CAS-Nr. 88-58-4), Phenothiazin (CAS-Nr. 92-84-2) und deren Derivaten besteht. Bevorzugt liegt der Stabilisator in einem Anteil von 0,01 bis 1 Gew.-% in der Komponente A vor.

Die Komponente A enthält ferner AV mindestens einen Akzelerator aus der Gruppe der aromatischen Amine. Bevorzugt handelt es sich bei dem Amin um ein Toluidin. Dabei ist der Akzelerator AV besonders bevorzugt aus der Gruppe ausgewählt, die aus N,N-Dimethyl-p-toluidin, N,N-Bis-hydroxyethyl-p-toluidin und N,N-Dimethyl-p-hydroxyethyl-anilin besteht. Der Gehalt des Akzelerators in der Komponenten A beträgt bevorzugt von 0,1 Gew.-% bis 4 Gew.-%.

In der Komponente A können ferner mit Vorteil ein oder mehrere optionale Zusatzstoffe enthalten sein, z.B. eine oder mehrere farbgebende Substanzen und/oder ein oder mehrere pharmazeutische Wirkstoffe.

Die pharmazeutischen Wirkstoffe können aus den aus den Gruppen der Antibiotika, Hormone, Wachstumsfaktoren und Antiphlogistika in der pastenförmigen Komponente A ausgewählt sein. Diese pharmazeutischen Wirkstoffe können im Monomer AI löslich oder auch unlöslich sein. Als pharmazeutische Wirkstoffe sind hierbei die Antibiotika Gentamicinsulfat, Tobramycinsulfat, Clindamycinhydrochlorid, Vancomycinhydrochlorid, Trometamol-Fosfomycin und Daptomycin bevorzugt.

In der Paste A können ferner ein oder mehrere Röntgenopaker enthalten sein, die auch als Röntgenkontrastmittel bezeichnet werden. Röntgen-opaker werden üblicherweise in Knochenzementen verwendet und sind im Handel erhältlich.

Bei dem Röntgenopaker kann es sich z.B. um Metalloxide, z.B. Zirkoniumdioxid, Bariumsulfat, toxikologisch unbedenkliche Schwer-metall-partikel, wie Tantal, Ferrit und Magnetit, oder toxikologisch unbedenkliche Calciumsalze, z.B. CaCO₃, CaSO₄ oder CaSO₄ ·2H₂O, handeln.

Bevorzugt ist der Röntgenopaker aus der Gruppe ausgewählt, die aus Zirkoniumdioxid, Bariumsulfat, Tantal und biokompatiblen Calciumsalzen besteht.

Weiterhin kann die Komponente A als Hilfsstoffe in Methacrylat lösliche Farbstoffe, in Methacrylat unlösliche Farbpigmente, pyrogenes Siliziumdioxid und Methacrylamid enthalten. Als in Methylacrylat löslicher Farbstoff ist besonders der Lebensmittelfarbstoff E141 (Chlorophyllin) geeignet.

Als nicht radikalisch polymerisierbare, bei Raumtemperatur und unter Normdruck flüssige Substanz BII werden Wasser, Glycerin (CAS-Nr. 56-81-5), Diglycerin (CAS-Nr. 627-82-7), Glycerin-tri-octoate (CAS-Nr.528-23-8), Glycerin-tri-decanoate (CAS-Nr. 621-71-6), Polyethylenglykol (CAS-Nr. 25322-68-3), Sebacinsäuredibutylester (CAS-Nr. 109-43-39und deren Gemische bevorzugt. In diesen Flüssigkeiten ist die Löslichkeit von Dibenzyolperoxid kleiner als 5 Gewichtsprozent bei Raumtemperatur. Besonders geeignet sind gemischte Glycerintriester der Octansäure und der Decansäure, die unter dem Markennamen Miglyol® 812 (CAS-Nr.52622-27-2) handelsüblich sind. Diese Flüssigkeit bildet bei Vermischung mit partikulärem Dibenzoylperoxid fließfähige Pasten, die auf Grund der Schmierwirkung des Miglyol® 812 sehr gut in Kartuschen mit kleinen Durchmessern bewegt werden können. Ebenso ist es möglich, polare Flüssigkeiten wie Wasser, Glycerin, Diglycerin und Polyethylenglykol zu verwenden. Wenn diese polaren Flüssigkeiten in der Komponente B enthalten sind, führen sie bei der Vermischung der Komponente A mit der Komponente B zu einer etwas verzögerten Aushärtung des gebildeten Zementteigs. Als Polyethylenglykol kommen Polyethylenglykol 200, Polyethylenglykol 400 und Polyethylenglykol 600 in Betracht.

Vorzugsweise enthält die Komponente B so gut wie keine bioverträglichen Polymerpulver, wie Homo- oder Copolymerisate von Acryl- und/oder Methacrylestern, Styrol, Vinyl-Derivaten oder deren Mischungen. Der Gehalt liegt bevorzugt bei weniger als 0,1 Gew.-%, besonders bevorzugt bei weniger als 0,01 Gew.-% und ganz besonders bei weniger als 0,001 Gew.-%.

Das Gewichtsverhältnis von Dibenzoylperoxid BI zur flüssigen Substanz BII ist größer, gleich 1:1 und bevorzugt größer, gleich 1:0,8 ist.

Das Gewichtsverhältnis von Dibenzoylperoxid BI zur flüssigen Substanz BII ist größer, gleich 1:1 und bevorzugt größer, gleich 1:0,8 ist.

Das Dibenzoylperoxid BI hat eine Korngröße der Siebfraktion kleiner, gleich 63 µm. Dadurch wird bei Vermischung der Komponente B mit der Komponente A eine gleichmäßige Verteilung des Dibenzoylperoxids erreicht.

Die pastenförmige Komponente A kann einer Sterilisation unterworfen werden, z.B. mit einem Sterilisationsmittel. Die Paste kann z.B. mit dem in EP 2 59 6812 A1 beschriebenen Sterilisationsverfahren durch Einwirkung von β-Propiolacton oder dessen Derivaten sterilisiert werden. Die Paste B kann entweder durch Gammabestrahlung, Elektronenbestrahlung oder durch Behandlung mit β-Propiolacton sterilisiert werden.

Erfindungsgemäß ist, dass die pastenförmigen Komponente A gemäß ISO Norm 5833 entsprechend dem "Doctors finger test" klebfrei ist. Dadurch ist gewährleistet, dass nach der Vermischung der Komponente A mit der Komponente B entstandene Zementteig sofort, ohne jegliche Wartezeit, klebfrei ist und damit sofort appliziert werden kann.

Die Erfindung betrifft auch ein Verfahren zur Herstellung eines selbsthärtenden Zementteigs mit dem erfindungsgemäßen Zweikomponenten-Polymethacrylat-Knochenzement, umfassend das Mischen der pastenförmigen Komponente A und der pastenförmigen Komponente B, um einen Zementteig zu bilden, der selbständig aushärtet. Die beiden Komponenten werden bevorzugt in einem Gewichtsverhältnis von 96 bis 99,9 Gewichtsteilen der Paste A zu 0,1 bis 4 Gewichtsteilen der Paste B vermischt.

Der Vermischungsvorgang der beiden Komponenten kann zeitlich vom Auspressvorgang getrennt werden. Die beiden Komponenten können z.B. in einer Kartusche, wie einer Kunststoffkartusche, vermischt werden, und der gebildete Zementteig mit einer Auspressvorrichtung ausgepresst werden. Hierfür eignen sich z.B. konventionelle, manuell angetriebene Auspressvorrichtungen, die auch bei konventionellen Zweikomponenten-Pasten-Knochenzementen eingesetzt werden.

Der erfindungsgemäße pastenförmige Zweikomponenten-Polymethacrylat-Knochenzement wird besonders vorteilhaft zur Herstellung eines Mittels zur Fixierung von Totalgelenkendoprothesen und Revisionsgelenkendoprothesen verwendet.

Weiterhin wird der pastenförmige Zweikomponenten-Polymethacrylat-Knochenzement zur Herstellung eines Mittels für die Vertebroplastie, für die Kyphoplastie, für die Femurhalsaugmentation und für die Herstellung von Spacern verwendet.

Der pastenförmige Zweikomponenten-Polymethacrylat-Knochenzement kann auch zur Herstellung von lokalen Wirkstofffreisetzungssystemen verwendet werden. Diese könne die Form von Kugeln, bohnenförmigen sphärischen Körpern, Stäben, Rohren und irregulär geformten Körpern haben.

Die Erfindung wird durch die nachfolgenden Beispiele 1-5 näher erläutert.

Herstellung der pastenförmigen Komponente A der Beispiele 1-5:
Die pastenförmigen Komponenten A der Beispiele 1 bis 5 wurden durch Vermischen der in Tabelle 1 angegebenen Bestandteile hergestellt.

**Tabelle 1:**

| | Zusammensetzung der pastenförmigen Komponente A | | | | |
|---|---|---|---|---|---|
| | Beispiel 1 | Beispiel 2 | Beispiel 3 | Beispiel 4 | Beispiel 5 |
| Methylmethacrylat | 42,50 g | 42,50 g | 42,50 g | 42,50 g | 42,50 g |
| Polymethylmethacrylat-co-methylacrylat | 30,50 g | 30,50 g | 30,50 g | 30,50 g | 30,50 g |
| vernetztes Polymethylmethacrylat | 15,45 g | 15,45 g | 15,45 g | 15,45 g | 15,45 g |
| Methacrylamid | 0,40 g | 0,40 g | 0,40 g | 0,40 g | 0,40 g |
| 2,5-Di-t-butylhydrochinon | 50 mg | 50 mg | 50 mg | 50 mg | 50 mg |
| N,N-Dimethyl-p-toluidin | 0,35 g | 0,35 g | 0,35 g | 0,35 g | 0,35 g |
| Grünlack | 5 mg | 5 mg | 5 mg | 5 mg | 5 mg |
| Gentamicinsulfat Aktivitätskoeffizient AK 580 | - | 1,47 | - | - | - |
| Clindamycinhydrochlorid | - | - | 1,50 | - | - |
| Vancomycinhydrochlorid | - | - | - | 1,50 | - |
| Trometamol-Fosfomycin | - | - | - | - | 1,50 |

Zusammensetzung der pastenförmigen Komponente B:
10,0 g 75%-iges BPO (phlegmatisiert mit 25 Gewichtsprozent Wasser)
4,0 g Mygliol

Die Komponenten der pastenförmigen Komponete B der Beispiele 1-5 wurden in Plastikdosen eingewogen, durch Rühren homogenisiert und anschließend mit einem Schraubdeckel luftdicht verschlossen. Die pastenförmigen Komponenten B wurden bei Raumtemperatur in den luftdicht verschlossenen Plastikdosen bis zu ihrer Verwendung gelagert.

Die beiden Komponenten A und B wurden miteinander vermahlen, bis eine farblose, cremigpastöse Masse entstanden war.

Die Substanzen der pastenförmigen Komponente A und B wurden von der Firma Sigma-Aldrich bezogen, bis auf das lösliche Polymethylmethacrylat-co-methylacrylat und das unlösliche Polymethylmethacrylat, die von Polymerherstellern zur Verfügung gestellt wurden. Das Trometamol-Fosfomycin wurde von der Firma ZaChem S.p.A. bezogen. Es wurde Gentamicinsulfat der Firma Fujian Fukang Ltd. (VR China) verwendet.

### Herstellung von Prüfkörpern:

Die ISO 5833 fordert eine Biegefestigkeit von ≥ 50 MPa, ein Biegemodul von ≥ 1800 MPa und eine Druckfestigkeit von ≥ 70 MPa. Zur Prüfung der mechanischen Eigenschaften gemäß der ISO 5833 der Pastenzemente der Beispiele 1-5 wurde Prüfkörper hergestellt. Es wurden 40 g Paste A wurde jeweils mit 1,40 g der pastenförmigen Komponente B der Beispiele 1-5 intensiv miteinander verrührt. Es entstand bei allen Beispielen ein sofort klebfreier, grüner Zementteig, der nach wenigen Minuten exotherm aushärtete. Es wurden mit dem Zementteig der Beispiele 1-5 jeweils streifenförmige Probekörper der Abmessungen 75 mm x 10 mm x 3,3 mm für die Prüfung der Biegefestigkeit und des Biegemoduls gemäß ISO5833 hergestellt. Weiterhin wurden zylinderförmige Probekörper (Durchmesser 6 mm, Höhe 12 mm) für die Prüfung der Druckfestigkeit gefertigt.

Nach Lagerung der Probekörper bei 23 °C, einer relativen Luftfeuchtigkeit von 50 %, über einen Zeitraum von 24 Stunden, wurden die Biegefestigkeit, das Biegemodul und die Druckfestigkeit gemäß ISO 5833 bestimmt. Die Ergebnisse in Tabelle 2 zeigen, dass die mechanischen Anforderungen der ISO5833 bezüglich der Biegefestigkeit, des Biegemoduls und der Druckfestigkeit von den Zementen der Beispiele 1-5 erfüllt wurden.

**Tabelle 2:**

| Beispiel | Biegefestigkeit | Biegemodul | Druckfestigkeit |
|---|---|---|---|
| | [MPa] | [MPa] | [MPa] |
| 1 | 65,3 ± 2,6 | 2651 ± 84 | 97,1 ± 3,2 |
| 2 | 62,8 ± 1,6 | 2651 ± 28 | 88,0 ± 2,6 |
| 3 | 64,6 ± 1,3 | 2712 ± 43 | 93,3 ± 3,7 |
| 4 | 62,1 ± 2,5 | 2637 ± 72 | 96,1 ± 3,0 |
| 5 | 63,2 ± 1,4 | 2575 ± 70 | 88,8 ± 1,8 |

### Beispiele 6-10

Die pastenförmige Komponente A und die pastenförmige Komponente B wurden in gleicher Weise wie bei den Beispielen 1-5 hergestellt, wobei jedoch jeweils 0,1 Gew.-% β-Propiolacton der pastenförmigen Komponente A und der pastenflörmigen Komponente B zugesetzt wurde. Die pastenförmige Komponente A und die pastenförmige Komponente B wurden 2 Wochen nach ihrer Herstellung und Lagerung bei Raumtemperatur im gleichen Mischungsverhältnis wie bei den Beispielen 1-5 manuell miteinander Vermischt. Es entstand, vergleichbar wie bei den Beispielen 1-5, ein grüner, klebfreier Zementteig, der ungefähr 3 Minuten und 30 Sekunden plastisch verformbar war und anschließend innerhalb von ca. 4 Minuten exotherm aushärtete.

### Beispiele 11-15

Die pastenförmige Komponente A und die pastenförmige Komponente B wurden in gleicher Weise wie bei den Beispielen 6-10 hergestellt, wobei jedoch jeweils 0,1 Gew.-% Propiolacton der pastenförmigen Komponente A und der pastenflörmigen Komponente B zugesetzt wurde. Die pastenförmige Komponente A und die pastenförmige Komponente B wurden 2 Wochen nach ihrer Herstellung und Lagerung bei Raumtemperatur im gleichen Mischungsverhältnis wie bei den Beispielen 1-5 in spezielle side-by-side-Kartuschen eingebracht. Anschließend wurden diese Kartuschen mit jeweils einem Austragsrohr und einem darin angeordneten statischen Mischer verbunden und dann mit einer manuell bedienbaren Auspressvorrichtung ausgepresst. Es entstand bei dem Auspressen, vergleichbar wie bei den Beispielen 1-5, ein sofort klebfreier, grüner Zementteig, der ungefähr 3 Minuten und 30 Sekunden plastisch verformbar war und anschließend innerhalb von ca. 4 Minuten exotherm aushärtete.

### Beispiele 16-20

Es wurde als pastenförmige Komponente B ein Gemisch aus 10,0 g 75%iges Dibenzoylperoxid (phlegmatisiert mit 25 Gewichtsprozent Wasser) und 4,0 g Glycerin verwendet. Die Zusammensetzung der pastenförmigen Komponente A war gleich wie in den Beispielen 1-5. Die pastenförmige Komponente A und die pastenförmige Komponente B wurden ein Tag nach ihrer Herstellung und Lagerung bei Raumtemperatur im gleichen Mischungsverhältnis wie bei den Beispielen 1-5 in spezielle side-by-side-Kartuschen eingebracht. Anschließend wurden diese Kartuschen mit jeweils einem Austragsrohr und einem darin angeordneten statischen Mischer verbunden und dann mit einer manuell bedienbaren Auspressvorrichtung ausgepresst. Es entstand bei dem Auspressen, vergleichbar wie bei den Beispielen 1-5, ein sofort klebfreier, grüner Zementteig, der ca. 4,5 Minuten plastisch verformbar war und anschließend innerhalb von ca. 4 Minuten exotherm aushärtete.

### Beispiele 21-25

Es wurde als pastenförmige Komponente B ein Gemisch aus 10,0g 75%iges Dibenzoylperoxid (phlegmatisiert mit 25 Gewichtsprozent Wasser) und 4,0 g Wasser verwendet. Die Zusammensetzung der pastenförmigen Komponente A war gleich wie in den Beispielen 1-5. Die pastenförmige Komponente A und die pastenförmige Komponente B wurden ein Tag nach ihrer Herstellung und Lagerung bei Raumtemperatur im gleichen Mischungsverhältnis wie bei den Beispielen 1-5 in spezielle side-by-side-Kartuschen eingebracht. Anschließend wurden diese Kartuschen mit jeweils einem Austragsrohr und einem darin angeordneten statischen Mischer verbunden und dann mit einer manuell bedienbaren Auspressvorrichtung ausgepresst. Es entstand bei dem Auspressen, vergleichbar wie bei den Beispielen 1-5, ein sofort klebfreier, grüner Zementteig, der 4 bis 5 Minuten plastisch verformbar war und anschließend innerhalb von ca. 4 Minuten exotherm aushärtete.

## Patentansprüche

1. Pastenförmiger Zweikomponentenpolymethacrylat-Knochenzement, umfassend eine pastenförmige Komponente A, enthaltend
AI mindestens ein destillierbares, radikalisch polymerisierbares Methacrylatmonomer,
AII mindestens ein in AI lösliches Polymer,
AIII mindestens ein in AI nicht lösliches partikuläres Polymer mit einer Partikelgröße von höchstens 500 µm,
AIV mindestens einen radikalischen Stabilisator und
AV mindestens einen Akzelerator aus der Gruppe der aromatischen Amine
und
eine pastenförmigen Komponente B, nur enthaltend
BI Dibenzoylperoxid,
BII mindestens eine nicht radikalisch polymerisierbare, bei Raumtemperatur flüssige Substanz, in der Dibenzoylperoxid eine Löslichkeit kleiner 5,0 Gewichtsprozent bei Raumtemperatur hat,
wobei nach Vermischung der pastenförmigen Komponente A mit der pastenförmigen Komponente B in dem gebildeten selbsthärtenden Zementteig, der Gewichtsanteil der flüssigen Substanz BII kleiner 2,0 Gewichtsprozent ist.

2. Pastenförmiger Zweikomponenten-Polymethacrylat-Knochenzement nach Anspruch 1,
**dadurch gekennzeichnet, dass**
die pastenförmige Komponente A enthält:
AI 20- 60 Gew.-% des mindestens einen destillierbaren, radikalisch polymerisierbaren Methacrylatmonomers,
AII 15-45 Gew.-% des mindestens einen in AI löslichen Polymer,
AIII 15-45 Gew.-% des mindestens einen in AI nicht löslichen partikulären Polymer mit einer Partikelgröße von höchstens 500 µm.

3. Pastenförmiger Zweikomponenten-Polymethacrylat-Knochenzement nach einem der vorstehenden Ansprüche **dadurch gekennzeichnet, dass** das Gewichtsverhältnis von Dibenzoylperoxid BI zur flüssigen Substanz BII größer, gleich 1:1 und bevorzugt größer gleich 2:1 ist.

4. Pastenförmiger Zweikomponenten-Polymethacrylat-Knochenzement nach einem der vorstehenden Ansprüche, **dadurch gekennzeichnet, dass**
das Gewichtsverhältnis der pastenförmigen Komponente A zur pastenförmigen Komponente B größer gleich 96 zu 4 ist.

5. Pastenförmiger Zweikomponenten-Polymethacrylat-Knochenzement nach einem der vorhergehenden Ansprüche, **dadurch gekennzeichnet, dass**
BII ausgewählt ist aus Wasser, Glycerin, Diglycerin, Glycerin-tri-octoate, Glycerin-tri-decanoate, Polyethylenglykol, Sebacinsäuredibutylester und deren Gemischen.

6. Pastenförmiger Zweikomponenten-Polymethacrylat-Knochenzement nach einem der vorstehenden Ansprüche **dadurch gekennzeichnet, dass**
BI Dibenzoylperoxid eine Korngröße der Siebfraktion kleiner, gleich 63 µm hat.

7. Pastenförmiger Zweikomponenten-Polymethacrylat-Knochenzement nach einem der vorstehenden Ansprüche **dadurch gekennzeichnet, dass** als Akzelerator AV N,N-Dimethyl-p-toluidin, N,N-Bis-hydroxyethyl-p-toluidin und N,N-Dimethyl-p-hydroxyethylanilin bevorzugt sind.

8. Pastenförmiger Zweikomponenten-Polymethacrylat-Knochenzement nach Anspruch 1 **dadurch gekennzeichnet, dass**
AI das Methacrylatmonomer aus der Gruppe ausgewählt ist, die aus Methylmethacrylat und Ethylenglykoldimethacrylat besteht.

9. Pastenförmiger Zweikomponenten-Polymethacrylat-Knochenzement nach einem der vorstehenden Ansprüche, **dadurch gekennzeichnet, dass**
Polymer AII aus der Gruppe der Polymethylmethacrylat-copolymere ausgewählt ist.

10. Pastenförmiger Zweikomponenten-Polymethacrylat-Knochenzement nach Anspruch 9, **dadurch gekennzeichnet, dass**
Polymer AII aus der Gruppe ausgewählt ist, die aus Polymethylmethacrylat-co-methylacrylat und Polymethylmethacrylat-co-styren besteht.

11. Pastenförmiger Zweikomponenten-Polymethacrylat-Knochenzement nach einem der vorstehenden Ansprüche **dadurch gekennzeichnet, dass**
AIII aus der Gruppe ausgewählt ist, die aus vernetztem Polymethylmethacrylat und vernetztem Polymethylmethacrylat-co-methylacrylat besteht.

12. Pastenförmiger Zweikomponenten-Polymethacrylat-Knochenzement nach einem der vorstehenden Ansprüche, **dadurch gekennzeichnet, dass**
der radikalische Stabilisator AIV aus der Gruppe ausgewählt ist, die aus p-Benzochinon, o-Benzochinon, 2,5-Di-t-butylhydrochinon, p-Hydrochinon und Phenothiazin besteht.

13. Pastenförmiger Zweikomponenten-Polymethacrylat-Knochenzement nach einem der vorstehenden Ansprüche, **dadurch gekennzeichnet, dass** die pastenförmige Komponente A als Hilfsstoffe eine oder mehrere Substanzen enthält, die aus der Gruppe ausgewählt sind, die aus in Methylmethacrylat löslichen Farbstoffen, in Methylmethacrylat unlöslichen Farbpigmentne, pyrogenem Siliziumdioxid und Methacrylamid besteht.

14. Pastenförmiger Zweikomponenten-Polymethacrylat-Knochenzement nach einem der vorstehenden Ansprüche **dadurch gekennzeichnet, dass**
die pastenförmige Komponente A einen oder mehrere pharmazeutische Wirkstoffe enthält, die aus der Gruppe ausgewählt sind, die Antibiotika, Hormone, Wachstumsfaktoren und Antiphlogistika besteht.

15. Pastenförmiger Zweikomponenten-Polymethacrylat-Knochenzement nach einem der vorstehenden Ansprüche, **dadurch gekennzeichnet, dass**
die pastenförmige Komponente A mindestens einen Röntgenopaker enthält, der aus der Gruppe ausgewählt ist, die aus Zirkoniumdioxid, Bariumsulfat, Tantal und biokompatiblen Calciumsalzen besteht.

16. Pastenförmiger Zweikomponenten-Polymethacrylat-Knochenzement nach einem der vorstehenden Ansprüche, **dadurch gekennzeichnet, dass**
in der pastenförmigen Komponente B ein Farbpigment suspendiert ist, das in der flüssigen Substanz BII unlöslich ist.

17. Verfahren zur Herstellung eines selbsthärtenden Knochenzementteigs mit dem Zweikomponenten-Polymethacrylat-Knochenzement nach einem der vorhergehenden Ansprüche **dadurch gekennzeichnet, dass** die pastenförmige Komponente A mit der pastenförmigen Komponente B in einem Gewichtsverhältnis größer gleich 96 zu 4 vermischt werden.

18. Verwendung des pastenförmigen Zweikomponenten-Polymethacrylat-Knochenzements nach einem der Ansprüche 1 bis 16 zur Herstellung eines Mittels zur Fixierung von Totalgelenkendoprothesen und Revisionsgelenkendoprothesen.

19. Verwendung eines pastenförmigen Zweikomponenten-Polymethacrylat-Knochenzements nach einem der Ansprüche 1 bis 16 zur Herstellung eines Mittels für die Vertebroplastie, für die Kyphoplastie, für die Femurhalsaugmentation und für die Herstellung von Spacern.

20. Verwendung des pastenförmigen Zweikomponenten-Polymethacrylat-Knochenzements nach einem der Ansprüche 1 bis 16 zur Herstellung von lokalen Wirkstofffreisetzungssystemen.

## Claims

1. A pasty two-component polymethacrylate bone cement, comprising a pasty component A, containing
AI at least one methacrylate monomer that can be distilled and is subject to radical polymerisation;
AII at least one polymer that is soluble in Al;
AIII at least one AI-insoluble particulate polymer with a particle size of at most 500 µm;
AIV at least one radical stabiliser; and
AV at least one accelerator from the group of the aromatic amines;
and
a pasty component B, only containing
BI dibenzoyl peroxide;
BII at least one substance that is not subject to radical polymerisation and is liquid at room temperature, in which the solubility of dibenzoyl peroxide at room temperature is less than 5.0% by weight,
whereby, after mixing pasty component A with pasty component B, the weight fraction of liquid substance BII in the self-curing cement dough thus formed is less than 2.0% by weight.

2. The pasty two-component polymethacrylate bone cement according to claim 1,
**characterised in that**
pasty component A contains:
AI 20-60% by weight of the at least one methacrylate monomer that can be distilled and is subject to radical polymerisation;
All 15-45% by weight of the at least one AI-soluble polymer;
AIII 15-45% by weight of the at least one AI-insoluble particulate polymer with a particle size of at most 500 µm.

3. The pasty two-component polymethacrylate bone cement according to any one of the preceding claims, **characterised in that** the weight ratio of dibenzoyl peroxide BI and liquid substance BII is larger than/equal to 1:1, and preferably is larger than/equal to 2:1.

4. The pasty two-component polymethacrylate bone cement according to any one of the preceding claims, **characterised in that**
the weight ratio of pasty component A and pasty component B is more than/equal to 96 to 4.

5. The pasty two-component polymethacrylate bone cement according to any one of the preceding claims, **characterised in that**
BII is selected from water, glycerol, diglycerol, glycerol-tri-octanoate, glycerol-tri-decanoate, polyethylene glycol, sebacic acid dibutyl ester, and mixtures thereof.

6. The pasty two-component polymethacrylate bone cement according to any one of the preceding claims, **characterised in that**
the grain size of the sieve fraction of BI dibenzoyl peroxide is less than/equal to 63 µm.

7. The pasty two-component polymethacrylate bone cement according to any one of the preceding claims, **characterised in that** N,N-dimethyl-p-toluidine, N,N-bis-hydroxyethyl-p-toluidine, and N,N-dimethyl-p-hydroxyethyl-aniline are preferred as accelerator AV.

8. The pasty two-component polymethacrylate bone cement according to claim 1,
**characterised in that**
AI, the methacrylate monomer, is selected from the group consisting of methyl methacrylate and ethylene glycol dimethacrylate.

9. The pasty two-component polymethacrylate bone cement according to any one of the preceding claims, **characterised in that**
polymer All is selected from the group of the polymethylmethacrylate copolymers.

10. The pasty two-component polymethacrylate bone cement according to claim 9,
**characterised in that**
polymer All is selected from the group consisting of polymethylmethacrylate-co-methyl acrylate and polymethylmethacrylate-co-styrene.

11. The pasty two-component polymethacrylate bone cement according to any one of the preceding claims, **characterised in that**
AIII is selected from the group consisting of cross-linked polymethylmethacrylate and cross-linked poly-methylmethacrylate-co-methyl acrylate.

12. The pasty two-component polymethacrylate bone cement according to any one of the preceding claims, **characterised in that**
radical stabilizer AIV is selected from the group consisting of p-benzoquinone, o-benzoquinone, 2,5-di-t-butylhydroquinone, p-hydroquinone, and phenothiazine.

13. The pasty two-component polymethacrylate bone cement according to any one of the preceding claims, **characterised in that** pasty component A contains, as excipients, one or more substances selected from the group consisting of methyl methacrylate-soluble dyes, methyl methacrylate-insoluble colour pigments, pyrogenic silicon dioxide, and methacrylamide.

14. The pasty two-component polymethacrylate bone cement according to any one of the preceding claims, **characterised in that**
pasty component A contains one or more pharmaceutical agents selected from the group consisting of antibiotics, hormones, growth factors, and antiphlogistic agents.

15. The pasty two-component polymethacrylate bone cement according to any one of the preceding claims, **characterised in that** pasty component A contains at least one radiopaquer selected from the group consisting of zirconium dioxide, barium sulfate, tantalum, and biocompatible calcium salts.

16. The pasty two-component polymethacrylate bone cement according to any one of the preceding claims, **characterised in that**
pasty component B has a colour pigment suspended in it that is insoluble in liquid substance BII.

17. A method for the production of a self-curing bone cement dough with the two-component poly-methacrylate bone cement according to any one of the preceding claims, **characterised in that**
pasty component A and pasty component B are being mixed at a weight ratio of more than/equal to 96 to 4.

18. A use of the pasty two component polymethacrylate bone cement according to any one of the claims 1 to 16 for production of a means for fixation of total articular endoprostheses and revision articular endoprostheses.

19. The use of a pasty two-component polymethacrylate bone cement according to any one of the claims 1 to 16 for production of a means for vertebroplasty, for kyphoplasty, for femoral neck augmentation, and for production of spacers.

20. The use of a pasty two-component polymethacrylate bone cement according to any one of the claims 1 to 16 for production of local agent release systems.

## Revendications

1. Ciment osseux de polyméthacrylate bicomposant pâteux, comprenant un composant pâteux A, contenant
AI au moins un monomère de méthacrylate polymérisable de manière radicalaire, pouvant être distillé,
AII au moins un polymère soluble dans AI,
AIII au moins un polymère particulaire non soluble dans AI, avec une taille de particule de maximum 500 µm,
AIV au moins un stabilisateur radicalaire et
AV au moins un accélérateur du groupe des amines aromatiques
et
un composant pâteux B, contenant seulement
BI du peroxyde de dibenzoyle,
BII au moins une substance liquide à température ambiante, non polymérisable de manière radicalaire, dans laquelle le peroxyde de dibenzoyle a une solubilité inférieure à 5,0% en poids à température ambiante,
le pourcentage en poids de la substance liquide BII étant inférieur à 2,0% en poids après le mélange du composant pâteux A avec le composant pâteux B dans la pâte de ciment auto-durcissante formée.

2. Ciment osseux de polyméthacrylate bicomposant pâteux conformément à la revendication n°1, **caractérisé en ce que**
le composant pâteux A contient :
AI de 20 à 60% en poids au moins d'un monomère de méthacrylate polymérisable de manière radicalaire, pouvant être distillé,
AII de 15 à 45% en poids au moins d'un polymère soluble dans AI,
AIII de 15 à 45% en poids au moins d'un polymère particulaire non soluble dans AI, avec une taille de particule de maximum 500 µm.

3. Ciment osseux de polyméthacrylate bicomposant pâteux conformément à l'une des revendications précédentes, **caractérisé en ce que**
le rapport du poids entre le peroxyde de dibenzoyle BI et la substance liquide BII est supérieur ou égal à 1:1 et de préférence supérieur ou égal à 2:1.

4. Ciment osseux de polyméthacrylate bicomposant pâteux conformément à l'une des revendications précédentes, **caractérisé en ce que**
le rapport du poids entre le composant pâteux A et le composant pâteux B est supérieur ou égal à 96:4.

5. Ciment osseux de polyméthacrylate bicomposant pâteux conformément à l'une des revendications précédentes, **caractérisé en ce que**
BII est sélectionné à partir d'eau, de glycérine, diglycérine, trioctoate de glycérine, tridécanoate de glycérine, polyéthylène glycol, dibutylester d'acide sébacique et de leurs mélanges.

6. Ciment osseux de polyméthacrylate bicomposant pâteux conformément à l'une des revendications précédentes, **caractérisé en ce que** BI le peroxyde de dibenzoyle a une granulométrie de la fraction tamisée inférieure ou égale à 63 µm.

7. Ciment osseux de polyméthacrylate bicomposant pâteux conformément à l'une des revendications précédentes, **caractérisé en ce que** le N,N-Diméthyl-p-toluidine, le N,N-Bis-hydroxéthyl-p-toluidine et le N,N-Diméthyl-p-hydroxéthylaniline sont privilégiés comme accélérateur AV.

8. Ciment osseux de polyméthacrylate bicomposant pâteux conformément à la revendication n°1, **caractérisé en ce que**
AI le monomère de méthacrylate est sélectionné dans le groupe qui se compose de méthacrylate de méthyle et de diméthacrylate d'éthylène glycol.

9. Ciment osseux de polyméthacrylate bicomposant pâteux conformément à l'une des revendications précédentes, **caractérisé en ce que**
le polymère AII est sélectionné dans le groupe des copolymères de polyméthacrylate de méthyle.

10. Ciment osseux de polyméthacrylate bicomposant pâteux conformément à la revendication n°9, **caractérisé en ce que** le polymère AII est sélectionné dans le groupe qui se compose de polyméthacrylate de méthyle-co-acrylate de méthyle et de polyméthacrylate de méthyle-co-styrène.

11. Ciment osseux de polyméthacrylate bicomposant pâteux conformément à l'une des revendications précédentes, **caractérisé en ce que**
AIII est sélectionné dans le groupe qui se compose de polyméthacrylate de méthyle réticulé et de polyméthacrylate de méthyle-co-acrylate de méthyle réticulé.

12. Ciment osseux de polyméthacrylate bicomposant pâteux conformément à l'une des revendications précédentes, **caractérisé en ce que**
le stabilisateur radicalaire AIV est sélectionné dans le groupe qui se compose de p-benzoquinone, d'o-benzoquinone, de 2,5-Di-t-butylhydroquinone, de p-hydroquinone et de phénothiazine.

13. Ciment osseux de polyméthacrylate bicomposant pâteux conformément à l'une des revendications précédentes, **caractérisé en ce que** le composant pâteux A contient, comme agents auxiliaires, une ou plusieurs substances qui sont sélectionnées dans le groupe qui se compose de colorants solubles dans le méthacrylate de méthyle, de pigments colorés insolubles dans le méthacrylate de méthyle, de dioxyde de silicium pyrogéné et de méthacrylamide.

14. Ciment osseux de polyméthacrylate bicomposant pâteux conformément à l'une des revendications précédentes, **caractérisé en ce que**
le composant pâteux A contient une ou plusieurs substances actives pharmaceutiques qui sont sélectionnées dans le groupe qui se compose d'antibiotiques, d'hormones, de facteurs de croissance et d'antiphlogistiques.

15. Ciment osseux de polyméthacrylate bicomposant pâteux conformément à l'une des revendications précédentes, **caractérisé en ce que** le composant pâteux A contient au moins un matériau radio-opaque qui est sélectionné dans le groupe qui se compose de dioxyde de zirkonium, de sulfate de baryum, de tantale et de sels de calcium biocompatibles.

16. Ciment osseux de polyméthacrylate bicomposant pâteux conformément à l'une des revendications précédentes, **caractérisé en ce que**
un pigment coloré qui est insoluble dans la substance liquide BII est suspendu dans le composant pâteux B.

17. Procédé pour la fabrication d'une pâte de ciment osseux auto-durcissante avec le ciment osseux de polyméthacrylate bicomposant conformément à l'une des revendications précédentes, **caractérisé en ce que**
le composant pâteux A est mélangé avec le composant pâteux B dans un rapport de poids supérieur ou égal à 96:4.

18. Utilisation du ciment osseux de polyméthacrylate bicomposant pâteux conformément à l'une des revendications n°1 à n°16 pour la fabrication d'un agent pour la fixation d'endoprothèses articulaires totales et d'endoprothèses articulaires de reprise.

19. Utilisation d'un ciment osseux de polyméthacrylate bicomposant pâteux conformément à l'une des revendications n°1 à n°16 pour la fabrication d'un agent pour la vertébroplastie, la kyphoplastie, l'augmentation du col du fémur et la fabrication d'espaceurs.

20. Utilisation du ciment osseux de polyméthacrylate bicomposant pâteux conformément à l'une des revendications n°1 à n°16 pour la fabrication de systèmes de libération de substances actives locales.
